# EUROPEAN PATENT APPLICATION

(11) **EP 4 700 370 A1**
(43) Date of publication of application: **25.02.2026**
(21) Application number: 24196220.8
(22) Date of filing: 23.08.2024
(51) Int. Cl.: G01N 21/64, G01N 15/1429

(54) **A COMPUTER-IMPLEMENTED METHOD FOR PROVIDING FLUORESCENT COMPOUND RECOMMENDATIONS FOR FLUORESCENT MARKING**

(71) Applicant: Danmarks Tekniske Universitet, 2800 Kongens Lyngby (DK)
(72) Inventor: Olsen, Lars Rønn, 2800 Kongens Lyngby (DK)
(74) Representative: Inspicos P/S

(57) **Abstract**

The present disclosure relates to a computer-implemented method for providing fluorescent compound recommendations for fluorescent marking. The method comprises the steps of: receiving digital representations of potential fluorescent compounds (4) for fluorescent marking; receiving apparatus-related parameters (3); providing respective spillover coefficients between respective pairs of the potential fluorescent compounds to obtain a set of spillover coefficients (8), wherein the respective spillover coefficients are calculated based on the apparatus-related parameters and are indicative of measurement spillover among the respective pairs of the potential fluorescent compounds; identifying minimum coefficients (9) constituting a subset of the set of spillover coefficients; identifying a subset of the potential fluorescent compounds (10), wherein each pair of compounds within the subset of the potential fluorescent compounds has a spillover coefficient among said minimum coefficients; and providing compounds of said subset of potential fluorescent compounds as the fluorescent compound recommendations (11). The present disclosure further relates to a corresponding data-processing system and a corresponding computer program.

## Description

### FIELD OF THE INVENTION

The present invention relates to a computer-implemented method for providing fluorescent compound recommendations for fluorescent marking. The invention further relates to a data processing system comprising at least one processor.

### BACKGROUND OF THE INVENTION

Fluorescent compounds are molecules which can be attached chemically to facilitate detection of a biomolecule, such as a protein, antibody, or amino acid. The fluorescent compound typically binds selectively to a specific region of functional group on the target biomolecule. As a result, the fluorescent compound enables light-based analytical methods, such as fluorescence imaging.

It is often desirable to utilize many different fluorescent compounds to perform complex analyses of biomolecule systems. Some modern apparatuses allow measurements of tens of fluorescent compounds simultaneously.

However, each fluorescent compound does not emit light at a specific narrow wavelength range but is instead associated with an emission spectrum. When having more than just a few fluorescent compounds, the emission spectra of the fluorescent compounds will tend to overlap significantly, making the signals from the compounds difficult to separate.

Thereby, a proper selection of fluorescent compounds becomes highly important for the quality of fluorescence measurements.

A large number of fluorescent compounds are presently available, and the emission spectra of these can overlap in various manners. This generally provides a large solution space of possible combinations of fluorescent compounds.

Identifying suitable combinations of fluorescent compounds can thereby become highly complex and time consuming. Even if a hypothetical optimal combination of fluorescent compounds is identified, this combination may not necessarily be desirable in view of the actual properties of the specific apparatus on which fluorescent measurements are to be performed.

Hence, there is a need for improved methods for providing fluorescent compound recommendations for fluorescent marking.

### SUMMARY OF THE INVENTION

On the above background, it is an object of preferred embodiments of the present disclosure to provide improved methods for providing fluorescent compound recommendations for fluorescent marking. Further, it is an object of preferred embodiments of the present disclosure to reduce negative influence from the properties of an apparatus on which fluorescent measurements are to be performed.

A first aspect of the present disclosure relates to a computer-implemented method for providing fluorescent compound recommendations for fluorescent marking, the method comprising the steps of:
receiving digital representations of potential fluorescent compounds for fluorescent marking;
receiving apparatus-related parameters related to light source emission spectra and/or wavelength-dependency of detection;
providing respective spillover coefficients between respective pairs of the potential fluorescent compounds to obtain a set of spillover coefficients, wherein the respective spillover coefficients are calculated based on the apparatus-related parameters and are indicative of measurement spillover among the respective pairs of the potential fluorescent compounds;
identifying minimum coefficients, the minimum coefficients constituting a subset of the set of spillover coefficients, each respective coefficient of the minimum coefficients being identified based on that respective coefficient being lower than other coefficients of the set of spillover coefficients;
identifying a subset of the potential fluorescent compounds, wherein each pair of compounds within the subset of the potential fluorescent compounds has a spillover coefficient among said minimum coefficients; and
providing at least some compounds of said subset of potential fluorescent compounds as the fluorescent compound recommendations.

The present method utilizes apparatus-related parameters related to light source emission spectra and/or wavelength-dependency of detection. Preferably, these apparatus-related parameters are indicative of properties of the apparatus upon which an eventual measurement based on the fluorescent compound recommendations is to be performed.

Based on the apparatus-related parameters, accurate spillover coefficients between respective pairs of potential fluorescent compounds can be provided. By employing these spillover coefficients to provide fluorescent compound recommendations, it may be possible to reduce the negative influence from the properties of the apparatus on which fluorescent measurements are to be performed

The computer-implemented method according to the present disclosure may for example be implemented on a computer system, such as a server connected to the internet, for example as a web application. Users can then access the application to receive fluorescent compound recommendations, for example based on the individual needs of users.

The method comprises receiving digital representations of potential fluorescent compounds for fluorescent marking. These may, for example, be provided by a user, by the computer system, or by a combination thereof. For example, the computer system may provide an initial list of potential fluorescent compounds which the user can then edit by removing and/or adding fluorescent compounds.

A user may have a preference towards certain fluorescent compounds, for example because the user already has these compounds available. On the other hand, a computer system may have access to a broader selection of compounds which, at least potentially, may yield a better combination of fluorescent compound recommendations. Thereby, input from both a user and a computer system can be advantageous.

The method also comprises receiving apparatus-related parameters. These may, for example, be provided by a user, by the computer system, or through a combination thereof. For example, the computer system may have pre-defined parameters of a number of commercially available apparatuses for measurements based on fluorescent compounds, and a user can then select the parameters associated with the relevant apparatus. Alternatively or additionally, a user can also manually provide the apparatus-related parameters.

The apparatus-related parameters may for example relate to light source emission spectra of the apparatus. An apparatus may have one or more light sources, with each of these light sources having a respective emission spectrum. The apparatus-related parameters may alternatively or additionally have a wavelength-dependency of detection. An apparatus may have one or more photodetectors, with each of these having a wavelength-dependent detection efficiency. Moreover, an apparatus may have one or more filters, each having a wavelength-dependent attenuation or selection. The wavelength-dependency of detection can then be indicative of the wavelength-dependent detection efficiency of the one or more photodetectors, and/or indicative of the wavelength-dependent attenuation of the one or more filters.

The method according to the present disclosure provides respective spillover coefficients between respective pairs of the potential fluorescent compounds. A spillover coefficient is indicative of measurement spillover among the respective pairs of the potential fluorescent compounds. Here, measurement spillover may generally be understood as a (risk of) detection of a signal from a one fluorescent compound in a measurement range or channel associated with measurement of another fluorescent compound.

Measurement spillover may also be referred to as fluorescence bleed-through.

The spillover coefficients are typically calculated based on the emission spectra of the potential fluorescent compounds and based on the apparatus-related parameters. Alternatively, they may also be predefined, such that for a given pair of fluorescent compounds (and apparatus-related parameters), the computer system does not have to re-calculate the spillover coefficient each time the method is to be executed.

Given the spillover coefficients, the minimum coefficients among these can be identified. These can typically be identified as the smallest coefficients among the set of spillover coefficients. In some examples, spillover coefficients are iteratively added to minimum coefficients, thereby iteratively increasing the size of this subset, for example until a subset of the potential fluorescent compounds have been identified, upon which the fluorescent compounds recommendations are based.

A subset of the potential fluorescent compounds can be identified based on the identified minimum coefficients. These minimum coefficients are indicative pairs of fluorescent compounds which have a low spillover, and which are thereby potentially well-suited for an eventual fluorescence measurement.

However, to provide an advantageous set of fluorescent compound recommendations, it is typically not sufficient that just two fluorescent compounds have a low spillover coefficient with respect to each other. These two fluorescent compounds should further preferably have a low spillover coefficient with respect to any other compound of the fluorescent compound recommendations. For a given set of fluorescent compounds, each spillover coefficient among a pair of compounds in this set which is not low will potentially decrease the quality of fluorescent measurements based on this set. Thus, when identifying a subset of the potential fluorescent compounds, each pair of compounds within this subset should preferably have a spillover coefficient among the minimum coefficients identifying previously. Thereby, the method can avoid the presence of pairs of compounds which to not have a low spillover coefficient.

Once a subset of the potential fluorescent compounds have been identified, in which each pair has a spillover coefficient among the minimum coefficients, the at least some compounds of the subset of potential fluorescent compounds are provided as the fluorescent compound recommendations, for example to a user via a user interface in communication with the computer system.

Thereby, the method according to the present disclosure can potentially provide fluorescent compound recommendations. Further, these recommendations can potentially reduce negative influence from the properties of an apparatus on which fluorescent measurements are performed.

Typically, at least some compounds of the subset of potential fluorescent compounds are provided as the fluorescent compound recommendations, or (all compounds of) the subset of potential fluorescent compounds are provided as the fluorescent compound recommendations.

In typical examples according to the present disclosure, spillover coefficients are iteratively identified to gradually increase the number of minimum coefficients. Upon adding an additional spillover coefficient, it is possible to check whether a subset of the potential fluorescent compounds can be identified. Generally, by identifying a subset of the potential fluorescent compounds which as restricted to pairs of compounds having a spillover coefficient among the minimum coefficients, the solution space can be reduced significantly, enabling efficient execution of the method.

Fluorescent compounds may alternatively be referred to as fluorescent tags, fluorescent labels, fluorescent probes, or fluorescent markers.

According to examples of the present disclosure, the wavelength-dependency of detection relate to one or more filters and/or one or more photodetectors.

For example, one or more filters and/or one or more photodetectors may define a detection range and/or a wavelength-dependent detection efficiency. Based on this, a spillover coefficient can be provided.

According to examples of the present disclosure, the light-source emission spectra and/or the wavelength-dependency of detection relate to a specific apparatus for flow cytometry, fluorescence imaging, or fluorescent microscopy.

By relating the light-source emission spectra and/or the wavelength-dependency of detection to a specific apparatus, it is possible to efficiently design a measurement strategy using that apparatus.

According to examples of the present disclosure, the potential fluorescent compounds are respectively associated with respective fluorescent compound emission spectra, wherein the respective spillover coefficients are calculated based on the apparatus-related parameters in combination with the respective fluorescent compound emission spectra.

According to examples of the present disclosure, the spillover coefficients are calculated based on evaluating the respective fluorescent compound emission spectra in combination with the wavelength-dependency of detection.

According to examples of the present disclosure, the respective fluorescent compound emission spectra are provided based on the light source emission spectra.

According to examples of the present disclosure, the potential fluorescent compounds are respectively associated with respective fluorescent compound absorption spectra, wherein the respective fluorescent compound emission spectra are provided based on the light source emission spectra in combination with the fluorescent compound absorption spectra.

Generally, by relating apparatus-related parameters to emission spectra and/or absorption spectra of fluorescent compounds, the practical accuracy of the calculated spillover coefficient may be improved which in turn can provide improved fluorescent compound recommendations.

According to examples of the present disclosure, said method comprises a step of using at least some compounds of the fluorescent compound recommendations in a measurement, for example a measurement in a biological system and/or in a measurement based on any of flow cytometry, fluorescence imaging, and fluorescent microscopy.

Hence, examples according to the present disclosure may extend beyond the provision of fluorescent compound recommendations via a computer.

A user may, based on receiving the recommendations, for example manually acquire the relevant fluorescent compounds to perform a measurement using an apparatus.

Preferably, the apparatus-related parameters represent properties of the apparatus on which said measurement is performed.

In some examples, at least some compounds of the fluorescent compound recommendations are used in a measurement, and in some examples, (all of) the fluorescent compound recommendations are used in a measurement.

According to examples of the present disclosure, the method comprises a step of receiving a user-provided compound number, wherein the number of compounds in the subset of the potential fluorescent compounds corresponds to the user-provided compound number.

It can be desirable for a user to specify a specific number of fluorescent compounds which is desired or required, for example for an intended future measurement. For example, a user can specify that seven fluorescent compounds are required.

In response, depending on exact implementation, minimum coefficients are identified to an extent which permits identification of a subset of fluorescent compounds in which the number of compounds corresponds to the user-provided compound number.

According to examples of the present disclosure, the method comprises a step of receiving user-provided pre-selected compounds among the potential fluorescent compounds, wherein the subset of the potential fluorescent compounds comprises at least some of the user-provided pre-selected compounds.

A user may have a preference towards certain fluorescent compounds, for example because these compounds are available, cheaper, or easier to use.

A preference towards such fluorescent compounds can be taken into account by indicating that these compounds are preferred through a step of receiving user-provided pre-selected compound among the potential fluorescent compounds.

In various example, a user can provide such pre-selected compounds as fixed criterion, in which the subset of potential fluorescent compounds comprises each of the user-provided pre-selected compounds.

In various examples, a user can provide pre-selected compounds as a relaxed criterion, in which the subset of potential fluorescent compounds comprises at least some of the pre-selected compounds. For example, a user can provide 10 different pre-selected compounds, and upon this, five compounds among these can be provided as fluorescent compound recommendations.

According to examples of the present disclosure, the step of identifying the subset of potential fluorescent compounds is performed by iteratively evaluating whether a group of potential fluorescent compounds exists in which each pair of compounds within this group of potential fluorescent compounds has a spillover coefficient among said minimum coefficients while additional coefficients are iteratively added to the minimum coefficients until such a group is identified to thereby provide the subset of the potential fluorescent compounds.

By iteratively evaluating whether a group of potential fluorescent compounds exists in which each pair of compounds within this group of potential fluorescent compounds gas a spillover coefficient among the minimum coefficients while additional coefficients are iteratively added to the minimum coefficients, it can be possible to efficiently identify improved fluorescent compound recommendations.

According to examples of the present disclosure, each of the potential fluorescent compounds represent a respective graph node of a network graph,
wherein each of the minimum coefficients represent a graph edge of the network graph,
wherein the subset of the potential fluorescent compounds is identified by identifying a graph clique in the network graph, wherein the potential fluorescent compounds representing graph nodes of said graph clique are selected as the subset of the potential fluorescent compounds.

A graph clique is a subset of a graph network in which each graph node of the subset is connected by a graph edge to each other graph node of the subset. In other words, every two distinct graph nodes of the graph clique are adjacent.

By identifying the subset of the potential fluorescent compounds through identification of a graph clique, it can be possible to efficiently identify improved fluorescent compound recommendations.

For example, n potential fluorescent compounds can be represented in a network graph having n graph nodes, each respective node representing a respective compound of the potential fluorescent compounds. Given a compound number k such as a user-provided compound number, a brute force algorithm can check whether the network graph contains a clique of size k in time O(n^{k}k²), i.e., in polynomial time, which can therefore be performed without requiring extensive computational resources.

According to examples of the present disclosure, the minimum coefficients are the smallest coefficients among the set of spillover coefficients.

A second aspect of the present disclosure relates to a data processing system comprising at least one processor configured to:
receive digital representations of potential fluorescent compounds for fluorescent marking;
receive apparatus-related parameters related to light source emission spectra and/or wavelength-dependency of detection;
provide respective spillover coefficients between respective pairs of the potential fluorescent compounds to obtain a set of spillover coefficients, wherein the respective spillover coefficients are calculated based on the apparatus-related parameters and are indicative of measurement spillover among the respective pairs of the potential fluorescent compounds;
identify minimum coefficients, the minimum coefficients constituting a subset of the set of spillover coefficients, each respective coefficient of the minimum coefficients being identified based on that respective coefficient being lower than other coefficients of the set of spillover coefficients;
identify a subset of the potential fluorescent compounds, wherein each pair of compounds within the subset of the potential fluorescent compounds has a spillover coefficient among said minimum coefficients; and
provide at least some compounds of said subset of potential fluorescent compounds as fluorescent compound recommendations.

A data processing system according to the second aspect may have any of the same or similar advantages and effects as provided by a method according to the first aspect.

In an alternative example, the data processing system further comprises at least one apparatus, wherein the apparatus-related parameters represent properties of said apparatus. Hence, an apparatus, such as an apparatus for flow cytometry, an apparatus for fluorescence imaging, or an apparatus for fluorescent microscopy, may be combined with a processor to form a single data processing system which is capable of both providing fluorescent compound recommendations and actually performing a measurement using at least some or all of these fluorescent compound recommendations.

A third aspect of the present disclosure relates to a computer program comprising instructions which, when the program is executed by a computer, cause the computer to carry out the steps of:
receiving digital representations of potential fluorescent compounds for fluorescent marking;
receiving apparatus-related parameters related to light source emission spectra and/or wavelength-dependency of detection;
providing respective spillover coefficients between respective pairs of the potential fluorescent compounds to obtain a set of spillover coefficients, wherein the respective spillover coefficients are calculated based on the apparatus-related parameters and are indicative of measurement spillover among the respective pairs of the potential fluorescent compounds;
identifying minimum coefficients, the minimum coefficients constituting a subset of the set of spillover coefficients, each respective coefficient of the minimum coefficients being identified based on that respective coefficient being lower than other coefficients of the set of spillover coefficients;
identifying a subset of the potential fluorescent compounds, wherein each pair of compounds within the subset of the potential fluorescent compounds has a spillover coefficient among said minimum coefficients; and
providing at least some compounds of said subset of potential fluorescent compounds as fluorescent compound recommendations.

A computer program according to the third aspect may have any of the same or similar advantages and effects as provided by a method according to the first aspect.

### BRIEF DESCRIPTION OF THE DRAWINGS

Embodiments of the invention will now be further described by reference to the accompanying drawings, in which:
Fig. 1 schematically illustrates a data processing system and a computer program according to an example of the present disclosure,
Fig. 2 illustrates an exemplary flow cytometry system for which fluorescent compound recommendations according to the present disclosure may be used,
Fig. 3 illustrates an example of emission spectra of fluorescent compounds,
Fig. 4a-d visually illustrate examples of calculations of spillover coefficients,
Fig. 5 illustrates method steps according to an example of the present disclosure, and
Fig. 6a-d illustrate method steps according to an example of the present disclosure in which fluorescent compounds are represented by graph nodes of a network graph.

### DETAILED DESCRIPTION

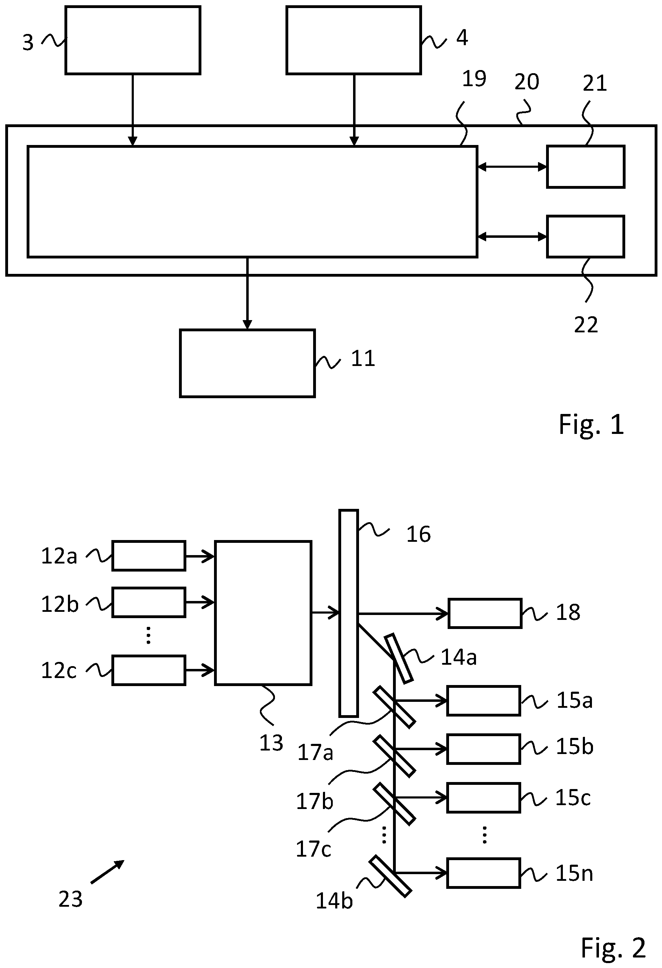

Fig. 1 schematically illustrates a data processing system 20 and a computer program 19 according to an example of the present disclosure.

The data processing system 20 comprises a processor 21 and a digital storage 22. The digital storage 22 comprises a storage medium for digitally recording and storing information. According to the present example, the digital storage 22 stores said computer program 19. In turn, the processor 21 is configured to execute said computer program 19.

In the present example, the data processing system 20 forms part of a server which is accessible by user through the internet. As input, a user can provide apparatus-related parameters 3 and potential fluorescent compounds 4, and based upon receiving this input, the data processing system 20 will, via the computer program 19, process these inputs to provide fluorescent compound recommendations 11 to the user.

The user can then use any or all of the fluorescent compound recommendations 11, for example in a subsequent measurement in a physical, biological system, such as a measurement based on flow cytometry, fluorescence imaging, or fluorescent microscopy.

By receiving fluorescent compound recommendations 11 from a data processing system 20 or a computer program 19 according to the present disclosure, an improved combination of fluorescent compounds may be provided.

The data processing system 20 and the computer program 19 are typically configured to provide fluorescent compound recommendations based on method steps or algorithmic steps as detailed in other examples of the present disclosure.

Fig. 2 illustrates an exemplary flow cytometry system 23 for which fluorescent compound recommendations according to the present disclosure may be used.

The system comprises a plurality of light sources 12a, 12b, ... 12c emitting light. In the figure, light propagation is represented by arrows. The light sources 12a, 12b, ... 12c may, for example, be light emitting diodes or lasers. By having a plurality of light sources, a broad spectrum of light can be emitted to thereby excite fluorescent compounds across this broad spectrum.

Light from the light sources 12a, 12b, ... 12c propagate into an optical system 13. The optical system 13 is configured to direct light from the various light sources 12a, 12b, ... 12c into a flow cell 16 of the system. The optical system 13 may, for example comprise one or more prisms, and/or one or more lenses.

Subsequent to the optical system 13, light enters the flow cell 16. In the flow cell, biomolecules flow with fluorescent compounds chemically attached. The fluorescent compounds are excited by light from the light sources 12a, 12b, ... 12c entering the cell 16, and correspondingly, fluorescent light is emitted from the fluorescent compounds.

Light from the light sources 12a, 12b, ... 12c not absorbed by the fluorescent compounds is detected by a forward scanner detector 18 arranged after the flow cell 16.

The fluorescent light emitted by the fluorescent compounds the exits the flow cell 16. In the present illustration, this light is redirect by a first mirror 14a towards a series of dichroic mirrors 17a, 17b, 17c, ..., which each redirect a portion of light to a respective detector 15a, 15b, 15c, .... After exiting the flow cell 16, the light may also be collected and collimated by one or more lenses, for example prior to propagating to the series of dichroic mirrors 17a, 17b, 17c, ....

In this particular example, each of the dichroic mirrors 17a, 17b, 17c, ... has a particular cutoff wavelength, where light having a wavelength above this cutoff wavelength is transmitted and light having a wavelength below this cutoff wavelength is reflected to be detected by a respective detector 15a, 15b, 15c, .... Thereby, these dichroic mirrors 17a, 17b, 17c, ... serve as wavelength filters. After the last dichroic mirror, a second mirror 14b is arranged, which redirects remaining light into a detector 15n.

As an example, a first dichroic mirror has a cutoff wavelength of 500 nm, a second dichroic mirror has a cutoff wavelength of 600 nm, a third dichroic mirror has a cutoff wavelength of 700 nm, and a fourth dichroic mirror has a cutoff wavelength of 800 nm. Thereby, the first dichroic mirror redirects light having a wavelength below 500 nm into a first detector, the second dichroic mirror redirects light having a wavelength between 500 nm and 600 nm into a second detector, the third dichroic mirror redirects light having a wavelength between 600 nm and 700 nm into a third detector, the fourth dichroic mirror redirects light having a wavelength between 700 nm and 800 nm into a fourth detector, and a final mirror redirects light having a wavelength above 800 nm into a fifth detector. These five detectors can thereby detect light emitted in respective wavelength ranges.

The present disclosure concerns to utilization of apparatus-related parameters related to light source emission spectra and/or wavelength-dependency of detection.

The flow cytometry system 23 illustrated in Fig. 2 comprises a plurality of light sources 12a, 12b, ... 12c. These light sources can define parameters related to light source emission spectra. Properties of the optical system 13 may also be taken into account for these parameters. Ideally, the parameters related to light source emission spectra should be indicative of the light which reaches the sample, which in the present case is the light which reaches the flow cell 16.

The flow cytometry system 23 further comprises a plurality of dichroic mirrors 17a, 17b, 17c, ... and a plurality of detectors 15a, 15b, 15c, .... 15n. These can define parameters related to wavelength-dependency of detection. For example, for the dichroic mirrors exemplified above, having cutoff frequencies at 500 nm, 600 nm, 700 nm, and 800 nm, light in the range below 500 nm is detected by the first detector 15a, thereby providing a first detection channel, light in the range from 500 nm to 600 nm is detected by the second detector 15b, thereby providing a second detection channel, etc.

A fluorescent compound having an emission spectrum which extends from, e.g., 400 nm to 520 nm may mostly be detected via the first detector 15a (or the first channel), but a certain portion of light may be detected via the second detector 15b (or the second channel), which constitutes spillover.

Note that examples according to the present disclosure are not generally limited to flow cytometry, or flow cytometry systems as the one exemplified in Fig. 2.

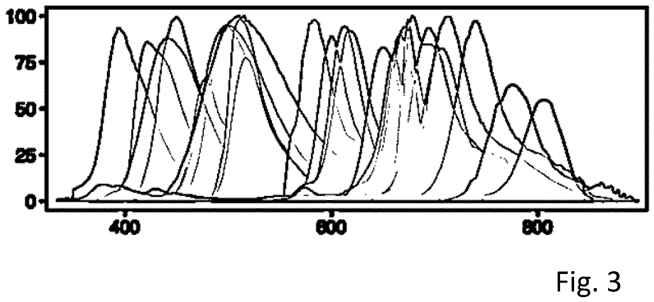

Fig. 3 illustrates an example of emission spectra of fluorescent compounds.

The horizontal axis indicates wavelength in units of nanometres and the vertical axis indicates a relative emission.

As evident from the figure, having many potential fluorescent compounds can provide a challenging starting point for a manual selection process. Such a selection process can be even more challenging considering apparatus-related parameters may influence which combination of fluorescent compounds which is optimal.

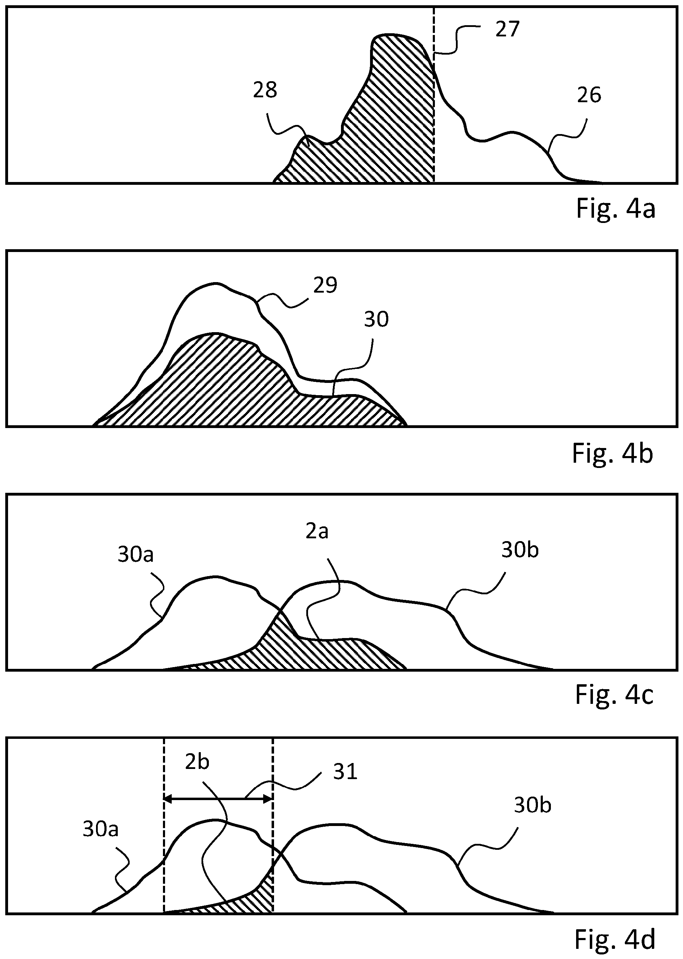

Fig. 4a-d visually illustrate examples of calculations of spillover coefficients. In each of the subfigures, the horizontal axis indicates wavelength and the vertical axis indicates a relative emission or absorption.

In Fig. 4a, an example of an absorption spectrum 26 of a fluorescent compound is illustrated.

As an example of apparatus-related parameters, parameters related to light source emission spectra can be provided. Such parameters may, for example, be one or more wavelengths indicative of laser wavelengths of a specific apparatus. These wavelengths thus determine which of various fluorescent compounds are excited, and to which degree.

In the present example, the absorption spectrum 26 is compared to these excitation wavelengths of, and the maximum wavelength within the spectrum 26 in which an excitation wavelength is present is identified. An example of such an identified excitation wavelength 27 is illustrated in Fig. 4a.

As a next step, an excitation fraction 28 is calculated by determining the area of the absorption spectrum 26 below the excitation wavelength relative the area of the entire absorption spectrum 26. In Fig. 4a, the excitation fraction is indicated by a shaded area.

Optionally, an excitation fraction may also take into account the presence of several excitation wavelengths, or take into account the magnitude of the absorption spectrum at which an excitation wavelength is present, thereby taking into account that a low magnitude can be indicative of a low absorption.

As an optional step, a fluorophore may be excluded from further consideration in case the excitation fraction is below a threshold, such as a predetermined threshold, for example set by a user. As an example, such a threshold is at most 0.5, for example at most 0.4, for example at most 0.3, such as at most 0.2.

In Fig. 4b, a theoretical emission spectrum 29 is illustrated. The emission spectrum is associated with the fluorescent compound for which an absorption spectrum 26 is illustrated in Fig. 4a. Based on the theoretical emission spectrum 29 and the excitation fraction 28, a calculated emission spectrum 30 is determined. Such a spectrum 30 may for example be calculated by multiplication of the theoretical emission spectrum 29 with the excitation fraction 28. For example, if the excitation fraction is 0.7, the calculated emission spectrum 30 may be obtained by multiplication of the theoretical emission spectrum 29 with 0.7.

Alternatively, the calculated emission spectrum 30 may also be determined by more complicated models of emission, for example a model taking into account the specific wavelengths at which a fluorescent compound is excited. Such a model may, for example, take into account that excitation at a low relative wavelength can shift emission towards a lower wavelength. Such a model may, for example, reduce the emission spectrum above an excitation wavelength more than below that excitation wavelength.

In Fig. 4c., a first calculated emission spectrum 30a and a second calculated emission spectrum 30b are shown. The first calculated emission spectrum 30a corresponds to the one illustrated in Fig. 4b, and the second calculated emission spectrum corresponds to that of another fluorescent compound.

Given these two emission spectra 30a, 30b, a spillover coefficient 2a can be calculated. In the present example, the spillover coefficient is calculated as a spectral overlap between the two emission spectra 30a, 30b as indicated by a shaded region in Fig. 4c. As an example, the spillover coefficient can be calculated as the Szymkiewicz-Simpson coefficient, which is provided as |A ∩ B|/min(|A, B|), where A and B can represent (the areas of) the two emission spectra 30a, 30b.

In Fig. 4d, another example of a calculation of a spillover coefficient 2b is provided. In this example, parameters related to wavelength-dependency of detection are taken into account. More specifically, a detection range 31 is provided. Such a detection range may for example be provided by dichroic mirror as exemplified in relation to Fig. 2, where mirrors and detectors provide a first detection range below 500 nm, a second detection range between 500 nm and 600 nm, a third detection range between 600 nm and 700 nm, etc.

In the present example, the detection range is taken into account by only including the portions of the emission spectra 30a, 30b which lie within the detection range when providing the spillover coefficient 2b. This is indicated in the illustration as a shaded region within the detection range. Generally, in case a wavelength-dependency of detection is provided as a wavelength-dependent efficiency, this efficiency can be multiplied with the relevant emission spectra prior to calculating the spillover coefficient between these spectra.

Optionally, several detection ranges may be of relevance to fluorescent compounds. In such cases, a spillover coefficient may be calculated with respect to each of these detection ranges. As an example, subsequent calculations may then take both of these into account when, e.g., identifying minimum coefficients, or subsequent calculations may be based on the greater of the two spillover coefficients.

Although absorption spectra can be utilized when providing spillover coefficients, it is also possible, within the scope of the present disclosure, to provide spillover coefficients without taking into account absorption spectra, for example by providing spillover coefficients based on emission spectra without taking into account, e.g., an excitation fraction.

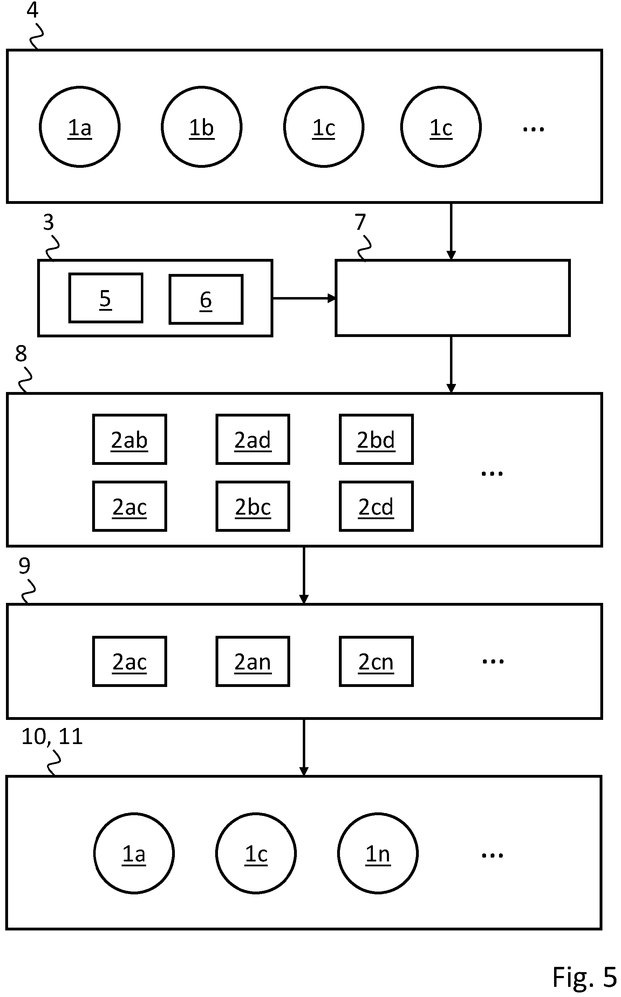

Fig. 5 illustrates method steps according to an example of the present disclosure.

As first steps, potential fluorescent compounds 4 and apparatus-related parameters 3 are received, for example based on user input to a data processing system.

In practice, a list of potential fluorescent compounds can for example be submitted by a user. Each fluorescent compounds can for example be associated with a molecular formula or illustration, an absorption spectrum, an emission spectrum, a commercial name, an identifier, a tag, or any combination thereof. Alternatively, the data processing system has available a list of potential fluorescent compounds. In Fig. 5, the compounds are labelled by reference numerals 1a, 1b, 1c, 1d, ..., corresponding to different fluorescent compounds.

The apparatus related parameters 3 can, for example, comprise parameters related to light source emission spectra 5, and parameters related to wavelength-dependency of detection 6.

As a next step, respective spillover coefficients 2ab, 2ac, 2ad, ... between pairs of potential fluorescence compounds are provided to obtain a set of spillover coefficients 8.

The spillover coefficients 2ab, 2ac, 2ad, ... are provided through a spillover coefficient calculation 7 which is based on the apparatus-related parameters 3 and the potential fluorescent compounds 4. The spillover coefficients 2ab, 2ac, 2ad, ... can be calculated based on the input provided, or, alternatively, pre-calculated and accessed by the data processing system. Examples of how spillover coefficients can be calculated are provided in relation to Figs. 4a-d.

Given n potential fluorescent compounds, the spillover coefficients 2ab, 2ac, 2ad, ... between each possible pair of fluorescent compounds 1a, 1b, 1c, 1d, ... amounts to n(n-1)/2 spillover coefficients. In the present illustration, the pair of fluorescent compounds labelled by 1a and 1b is associated with the spillover coefficient labelled 2ab, the pair of fluorescent compounds labelled by 1a and 1c is associated with the spillover coefficient labelled 2ac, etc.

Given the set of spillover coefficients 8, minimum coefficients 9 are identified. The minimum coefficients 9 correspond to the smallest coefficients among the set of spillover coefficients 8, and they thereby constitute a subset of the set of spillover coefficients 8.

Next, a subset of the potential fluorescent compounds 10 is identified based on the minimum coefficients 9. Each pair of fluorescent compounds 1a, 1c, 1n, ... within this subset of potential fluorescent compounds 10 is associated with a spillover coefficient among the minimum coefficients 9.

In the present example the minimum coefficients 9 comprises the spillover coefficients labelled with reference numerals 2ac, 2an, and 2cn, and correspondingly, the identified subset of potential fluorescent compounds 10 comprises the fluorescent compounds associated with these minimum coefficients 9, i.e., the fluorescent compounds labelled with reference numerals 1a, 1c, and 1n.

Finally, at least some compounds of the subset of potential fluorescent compounds 10, typically all of these compounds, are provided as fluorescent compound recommendations 11, for example to a user.

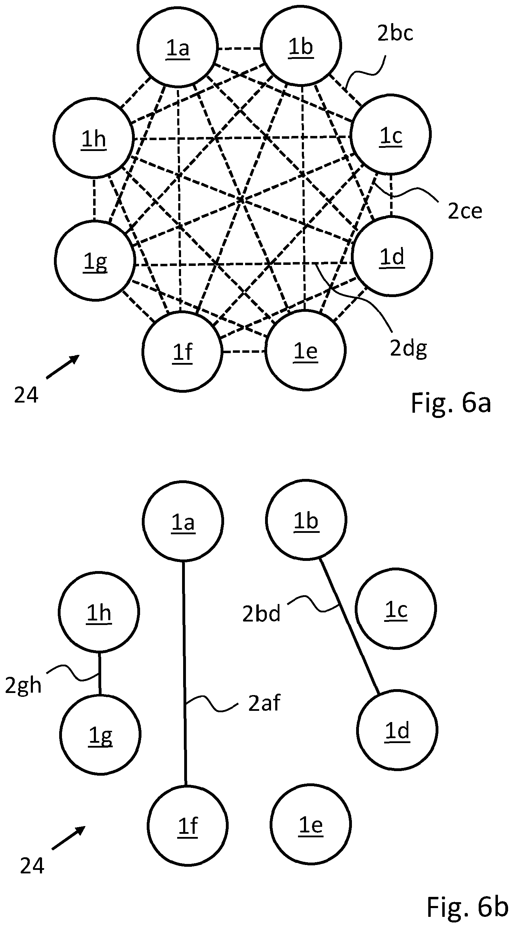

Fig. 6a-d illustrate algorithmic steps according to an example of the present disclosure in which fluorescent compounds are represented by graph nodes of a network graph 25.

In the present example, eight fluorescent compounds 1a, 1b, ... 1h are provided as potential fluorescent compounds. As illustrated in Fig. 6a, each of these can be considered to represent a respective graph node of a network graph 24. Further, spillover coefficients 2bc, 2ce, 2dg can be considered to represent graph edges, as also illustrated in Fig. 6a, in which each possible graph edge is shown.

Hence, the illustration shown in Fig. 6a may be considered to represent the potential fluorescent compounds and the provided respective spillover coefficients between the respective pairs of the potential fluorescent compounds. Since each pair of fluorescent compounds is associated with a spillover coefficient, a mapping or representation of these elements via a network graph 24 is well-suited.

As a next algorithmic step, the potential fluorescent compounds are considered in a network graph 24 in which graph edges representing minimum coefficients are iteratively added. A scenario in which three minimum coefficients have been identified, and corresponding graph edged have been added, is illustrated in Fig. 6b.

In typical implementations, all the spillover coefficients (represented by all graph edges in Fig. 6a) are sorted according to size, and graph edges are added one at a time according to this sorting.

The network graph 24 is iteratively analysed to identify whether a graph clique is formed as graph edges based on minimum coefficients are iteratively added. A graph clique is a subset of the graph network in which each graph node of this subset is connected by a graph edge to each other graph node of the subset.

In typical implementations, the network graph 24 is analysed to identify whether a graph clique is formed each time an additional graph edge, corresponding to an additional minimum coefficient, is added.

In the present example, a compound number of four has been provided by a user, and accordingly, the network graph is iteratively analysed to identify whether a graph clique of size four has been formed. In Fig. 6b, this is not the case. Accordingly, further graph edges, corresponding to minimum coefficients, are iteratively added to the network graph 24.

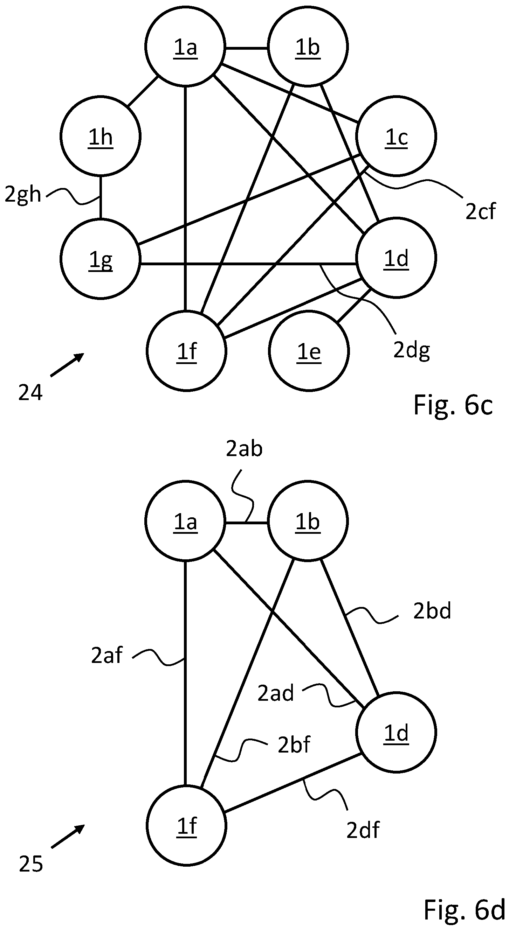

In Fig. 6c, such further graph edges have been added to the network graph 24 in comparison to the network graph in Fig. 6b. As a result, a graph clique of size four is present in Fig. 6c. The graph nodes (representing fluorescent compounds) of this graph clique are labelled with reference numerals1a, 1b, 1d, and 1f.

Fig. 6d illustrates, in isolation, the graph clique 25 present in the network graph 24 of Fig. 6c. Within the graph clique 25, each graph node is connected by a graph edge to each other graph node.

In terms of fluorescent compounds and spillover coefficients, the fluorescent compounds 1a, 1b, 1d, 1f represented by graph nodes of the graph clique 25 each have relatively small spillover coefficients, since the graph edges of the graph clique has been added based on being small relative to other spillover coefficients.

Finally, these fluorescent compounds 1a, 1b, 1d, 1f are provided as fluorescent compound recommendations to a user.

Thereby, these fluorescent compounds can be utilized in measurements to reduce negative influence, in particular spillover, from the properties of the apparatus on which the measurements are performed.

In case a user has pre-selected one or more compounds among the potential fluorescent compounds, a graph network can be analysed to identify whether a graph clique is formed which comprises respective graph nodes representing each of the one or more pre-selected compounds. In other words, a graph clique which does not comprise the one or more pre-selected compounds are ignored. Once a graph clique comprising the one or more pre-selected compounds is identified, the fluorescent compounds represented by graph nodes of this graph clique can be provided as fluorescent compound recommendations.

### List of figure references:

- 1: fluorescent compound
- 2: spillover coefficient
- 3: apparatus-related parameters
- 4: potential fluorescent compounds
- 5: parameters related to light source emission spectra
- 6: parameters related to wavelength-dependency of detection
- 7: spillover coefficient calculation
- 8: set of spillover coefficients
- 9: minimum coefficients
- 10: subset of potential fluorescent compounds
- 11: fluorescent compound recommendations
- 12: light source
- 13: optical system
- 14: mirror
- 15: detector
- 16: flow cell
- 17: dichroic mirror
- 18: forward scanner detector
- 19: computer program
- 20: data processing system
- 21: processor
- 22: digital storage
- 23: flow cytometry system
- 24: network graph
- 25: graph clique
- 26: absorption spectrum
- 27: excitation wavelength
- 28: excitation fraction
- 29: theoretical emission spectrum
- 30: calculated emission spectrum
- 31: detection range

## Claims

1. A computer-implemented method for providing fluorescent compound recommendations for fluorescent marking, the method comprising the steps of:
receiving digital representations of potential fluorescent compounds for fluorescent marking;
receiving apparatus-related parameters related to light source emission spectra and/or wavelength-dependency of detection;
providing respective spillover coefficients between respective pairs of the potential fluorescent compounds to obtain a set of spillover coefficients, wherein
the respective spillover coefficients are calculated based on the apparatus-related parameters and are indicative of measurement spillover among the respective pairs of the potential fluorescent compounds;
identifying minimum coefficients, the minimum coefficients constituting a subset of the set of spillover coefficients, each respective coefficient of the minimum coefficients being identified based on that respective coefficient being lower than other coefficients of the set of spillover coefficients;
identifying a subset of the potential fluorescent compounds, wherein each pair of compounds within the subset of the potential fluorescent compounds has a spillover coefficient among said minimum coefficients; and
providing at least some compounds of said subset of potential fluorescent compounds as the fluorescent compound recommendations.

2. A method according to claim 1, wherein the wavelength-dependency of detection relate to one or more filters and/or one or more photodetectors.

3. A method according to any of the preceding claims, wherein the light-source emission spectra and/or the wavelength-dependency of detection relate to a specific apparatus for flow cytometry, fluorescence imaging, or fluorescent microscopy.

4. A method according to any of the preceding claims, wherein the potential fluorescent compounds are respectively associated with respective fluorescent compound emission spectra, wherein the respective spillover coefficients are calculated based on the apparatus-related parameters in combination with the respective fluorescent compound emission spectra.

5. A method according to claim 4, wherein the spillover coefficients are calculated based on evaluating the respective fluorescent compound emission spectra in combination with the wavelength-dependency of detection.

6. A method according to any of claims 4-5, wherein the respective fluorescent compound emission spectra are provided based on the light source emission spectra.

7. A method according to any of claims 4-6, wherein the potential fluorescent compounds are respectively associated with respective fluorescent compound absorption spectra, wherein the respective fluorescent compound emission spectra are provided based on the light source emission spectra in combination with the fluorescent compound absorption spectra.

8. A method according to any of the preceding claims, wherein said method comprises a step of using at least some compounds of the fluorescent compound recommendations in a measurement, for example a measurement in a biological system and/or in a measurement based on any of flow cytometry, fluorescence imaging, and fluorescent microscopy.

9. A method according to any of the preceding claims, wherein the method comprises a step of receiving a user-provided compound number, wherein the number of compounds in the subset of the potential fluorescent compounds corresponds to the user-provided compound number.

10. A method according to any of the preceding claims, wherein the method comprises a step of receiving user-provided pre-selected compounds among the potential fluorescent compounds, wherein the subset of the potential fluorescent compounds comprises at least some of the user-provided pre-selected compounds.

11. A method according to any of the preceding claims, wherein the step of identifying the subset of potential fluorescent compounds is performed by iteratively evaluating whether a group of potential fluorescent compounds exists in which each pair of compounds within this group of potential fluorescent compounds has a spillover coefficient among said minimum coefficients while additional coefficients are iteratively added to the minimum coefficients until such a group is identified to thereby provide the subset of the potential fluorescent compounds.

12. A method according to any of the preceding claims, wherein each of the potential fluorescent compounds represent a respective graph node of a network graph,
wherein each of the minimum coefficients represent a graph edge of the network graph,
wherein the subset of the potential fluorescent compounds is identified by identifying a graph clique in the network graph, wherein the potential fluorescent compounds representing graph nodes of said graph clique are selected as the subset of the potential fluorescent compounds.

13. A method according to any of the preceding claims, wherein the minimum coefficients are the smallest coefficients among the set of spillover coefficients.

14. A data processing system comprising at least one processor configured to:
receive digital representations of potential fluorescent compounds for fluorescent marking;
receive apparatus-related parameters related to light source emission spectra and/or wavelength-dependency of detection;
provide respective spillover coefficients between respective pairs of the potential fluorescent compounds to obtain a set of spillover coefficients, wherein
the respective spillover coefficients are calculated based on the apparatus-related parameters and are indicative of measurement spillover among the respective pairs of the potential fluorescent compounds;
identify minimum coefficients, the minimum coefficients constituting a subset of the set of spillover coefficients, each respective coefficient of the minimum coefficients being identified based on that respective coefficient being lower than other coefficients of the set of spillover coefficients;
identify a subset of the potential fluorescent compounds, wherein each pair of compounds within the subset of the potential fluorescent compounds has a spillover coefficient among said minimum coefficients; and
provide at least some compounds of said subset of potential fluorescent compounds as fluorescent compound recommendations.

15. A computer program comprising instructions which, when the program is executed by a computer, cause the computer to carry out the steps of:
receiving digital representations of potential fluorescent compounds for fluorescent marking;
receiving apparatus-related parameters related to light source emission spectra and/or wavelength-dependency of detection;
providing respective spillover coefficients between respective pairs of the potential fluorescent compounds to obtain a set of spillover coefficients, wherein
the respective spillover coefficients are calculated based on the apparatus-related parameters and are indicative of measurement spillover among the respective pairs of the potential fluorescent compounds;
identifying minimum coefficients, the minimum coefficients constituting a subset of the set of spillover coefficients, each respective coefficient of the minimum coefficients being identified based on that respective coefficient being lower than other coefficients of the set of spillover coefficients;
identifying a subset of the potential fluorescent compounds, wherein each pair of compounds within the subset of the potential fluorescent compounds has a spillover coefficient among said minimum coefficients; and
providing at least some compounds of said subset of potential fluorescent compounds as fluorescent compound recommendations.
